(19) Europäisches Patentamt
European Patent Office
Office européen des brevets

(11) Veröffentlichungsnummer: **0 317 505**
**A1**

# (12) EUROPÄISCHE PATENTANMELDUNG

(21) Anmeldenummer: **88810751.3**

(22) Anmeldetag: **03.11.88**

(51) Int. Cl.4: **C 07 F 9/65**
A 61 K 31/675

(30) Priorität: **13.11.87 CH 4435/87**

(43) Veröffentlichungstag der Anmeldung:
**24.05.89 Patentblatt 89/21**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Jaeggi, Knut A., Dr.**
**General Guisan-Strasse 44**
**CH-4054 Basel (CH)**

Patentansprüche für folgende Vertragsstaaten: ES + GR.

(54) **Neue Azacycloalkylalkandiphosphonsäuren.**

(57) Azacycloalkylalkandiphosphonsäuren der Formel

$$R - alk - \underset{PO_3H_2}{\overset{PO_3H_2}{C}} - OH \qquad (I)$$

worin R einen über das Azaringglied gebundenen, aus 3- bis und mit 8-gliedrigen Ringsystemen aufgebaute Aza-bicycloalkylrest und alk Niederalkylen darstellt, und ihre Salze haben regulierende Eigenschaften auf den Calciumstoffwechsel und können als Arzneimittel zur Behandlung von Erkrankungen, die mit Störungen des Calciumstoffwechsels in Verbindung gebracht werden können, verwendet werden. Sie werden beispielsweise hergestellt, indem man in einer Verbindung der Formel

$$R - alk - \underset{X_2}{\overset{X_1}{C}} - OH \qquad (II),$$

worin $X_1$ eine funktionell abgewandelte und $X_2$ eine freie oder funktionell abgewandelte Phosphonogruppe bedeutet, $X_1$ und gegebenenfalls $X_2$ in die freie Phosphonogruppe überführt.

EP 0 317 505 A1

**Beschreibung**

## Neue Azacycloalkylalkandiphosphonsäuren

Die Erfindung betrifft neue Azacycloalkylalkandiphosphonsäuren der Formel

$$R - alk - \begin{array}{c} PO_3H_2 \\ | \\ C - OH \\ | \\ PO_3H_2 \end{array} \qquad (I)$$

worin R einen über das Azaringglied gebundenen, aus 3- bis und mit 8-gliedrigen Ringsystemen aufgebaute Azabicycloalkylrest bedeutet und alk Niederalkylen darstellt, und ihre Salze.

Ueber das Azaringglied gebundene, aus 3- bis und mit 8-gliedrigen Ringsystemen aufgebaute Azabicycloalkylreste sind insbesondere aus 4-bis 8-gliedrigen Ringsystemen aufgebaut und stellen beispielsweise über das Azaringglied gebundene, gegebenenfalls durch bis und mit 3 Niederalkylgruppen substituierte Aza-$C_6$-$C_{10}$-bicycloalkylreste, wie 3-Aza-bicyclo-$C_6$-$C_{10}$-alk-3-ylreste, z.B. 3-Aza-bicyclo[3,1,0]hex-3-yl bzw. 1,5-Dimethyl-3-aza-bicyclo[3,1,0]hex-3-yl, 3-Aza-bicyclo [3,2,0]hept-3-yl bzw. 1,5-Dimethyl-3-aza-bicyclo[3,2,0]hept-3-yl, 3-Aza-bicyclo[3,1,1]hept-3-yl bzw. 1,5-Dimethyl-3-aza-bicyclo[3,1,1]hept-3-yl, 7-Aza-bicyclo[2.2.1]hept-7-yl, 2-Aza-bicyclo[3,2,1]oct-2-yl, 3-Aza-bicyclo[3,2,1]oct-3-yl, 8-Aza-bicyclo[3.2.1]oct-8-yl, 3-Aza-bicyclo[3,2,2]non-3-yl oder 3-Aza-bicyclo[4,2,2]dec-3-yl, dar.

Nachstehend sind unter niederen Resten und Verbindungen beispielsweise solche zu verstehen, die bis und mit 7, insbesondere bis und mit 4, C-Atome aufweisen. Ferner haben die Allgemeinbegriffe beispielsweise folgende Bedeutungen:

Niederalkyl ist beispielsweise $C_1$-$C_4$-Alkyl, wie Methyl, Aethyl, Propyl, Isopropyl, Butyl, Isobutyl, Sekundär- oder Tertiärbutyl, kann aber auch eine $C_5$-$C_7$-Alkyl, d.h. Pentyl-, Hexyl- oder Heptylgruppe, sein.

Niederalkylen ist beispielsweise $C_1$-$C_7$, insbesondere $C_1$-$C_4$-Alkylen, vorzugsweise $C_2$-$C_4$-Alkylen wie Aethylen, 1,2- oder 1,3-Propylen, 1,2-, 1,3- oder 1,4-Butylen oder 1,2-(2-Methyl)propylen, kann aber auch 1,4-oder 1,5-Pentylen, 1,3-(3-Methyl)butylen, 1,2-(2-Ethyl)butylen oder 1,4-(4-Methyl)pentylen bedeuten.

Salze von Verbindungen der Formel I sind insbesondere deren Salze mit pharmazeutisch verwendbaren Basen, wie nicht-toxische, von Metallen der Gruppen Ia, Ib, IIa und IIb abgeleitete Metallsalze, z.B. Alkalimetall-, insbesondere Natrium- oder Kaliumsalze, Erdalkalimetall-, insbesondere Calcium- oder Magnesiumsalze, Kupfer-, Aluminium- oder Zinksalze, ebenso Ammoniumsalze mit Ammoniak oder organischen Aminen oder quaternären Ammoniumbasen, wie gegebenenfalls C-hydroxylierten aliphatischen Aminen, insbesondere Mono-, Di-oder Triniederalkylaminen, z.B. Methyl-, Ethyl-, Dimethyl- oder Diethylamin, Mono-, Di- oder Tri-(hydroxyniederalyl)aminen, wie Ethanol-, Diethanol- oder Triethanolamin, Tris(hydroxymethyl)-amino-methan oder 2-Hydroxytertiärbutylamin, oder N-(Hydroxyniederalkyl-N,N-diniederalkylaminen bzw. N-(Polyhydroxyniederalkyl-N-niederalkylaminen, wie 2-(Dimethylamino)-ethanol oder D-Glucamin, oder quaternären aliphatischen Ammoniumhydroxiden, z.B. mit Tetrabutylammoniumhydroxid.

Die Verbindungen der Formel I und ihre Salze weisen wertvolle pharmakologische Eigenschaften auf. Insbesondere besitzen sie eine ausgeprägte regulierende Wirkung auf den Calcium-Stoffwechsel von Warmblütern. Insbesondere bewirken sie an der Ratte eine ausgeprägte Hemmung der Knochenresorption, die sich sowohl in der Versuchsanordnung gemäss Acta Endocrinol. 78, 613-24 (1975) anhand des PTH-induzierten Anstieges des Serumcalciumspiegels nach subcutaner Applikation in Dosen von etwa 0,01 bis etwa 1,0 mg/kg, als auch im TPTX (Thyroparathyroidectomised) -Rattenmodell anhand der durch Vitamin $D_3$ ausgelösten experimentellen Hypercalcämie nach Gabe von Dosen von etwa 0,001 bis 1.0 mg s.c. zeigen lässt. Ebenso wird die durch Walker-256-Tumore induzierte Tumorhyper kalzämie nach peroraler Verabreichung von etwa 1,0 bis etwa 100 mg/kg gehemmt. Ferner zeigen sie in der Adjuvansarthritis der Ratte in der Versuchsanordnung nach Newbould, Brit. J. Pharmacology 21, 127 (1963) sowie nach Kaibara et al., J. Exp. Med. 159, 1388-96 (1984) in Dosen von etwa 0,01 bis 1,0 mg/kg s.c. eine deutliche Hemmung auf das Fortschreiten chronisch-arthritischer Prozesse. Sie sind deshalb vorzüglich geeignet als Arzneimittelwirkstoffe für die Behandlung von Erkrankungen, die mit Störungen des Calciumstoffwechsels in Verbindung gebracht werden können, beispielsweise entzündlicher Prozesse in Gelenken, degenerativer Prozesse im Gelenkknorpel, von Osteoporosis, Periodontitis, Hyperparathyreoidismus und von Calciumablagerungen in Blutgefässen oder an prothetischen Implantaten. Günstig beeinflusst werden sowohl Erkrankungen, bei denen eine anomale Ablagerung schwerlöslicher Calciumsalze festzustellen ist, wie solcher aus den Formenkreisen der Arthritis, z.B. Morbus Bechterew, der Neuritis, der Bursitis, Periodontitis und der Tendinitis, der Fibrodysplasie, der Osteoarthrose oder der Artereosklerose, als auch solche, bei denen eine anomale Auflösung harter Körpergewebe im Vordergrund steht, wie die hereditäre Hypophosphatasie, degenerative Prozesse im Gelenkknorpel, Osteoporosen verschiedener Genese, morbus Paget und die Osteodystrophia fibrosa, ebenso durch Tumore ausgelöste osteolytische Prozesse.

Die Erfindung betrifft in erster Linie Verbindungen der Formel I, worin R einen über das Azaringglied gebundenen, aus 3- bis und mit 8-, insbesondere 4- bis 7-gliedrigen Ringsystemen aufgebauten Aza-bicyclo-$C_6$-$C_{10}$-alkylrest bedeutet und alk $C_1$-$C_7$-, insbesondere $C_1$-$C_4$-Alkylen, darstellt, und ihre Salze, insbesondere ihre pharmazeutisch verwendbaren Salze mit Basen.

Die Erfindung betrifft vor allem Verbindungen der

Formel I, worin R einen über das Azaringglied gebundenen, aus 4- bis 7-gliedrigen Ringsystemen aufgebauten Aza-bicyclo-$C_6$-$C_{10}$-alkylrest, wie 3-Aza-bicyclo[3,1,0]hex-3-yl bzw. 1,5-Dimethyl-3-aza-bicyclo[3,1,0]hex-3-yl, 3-Aza-bicyclo[3,2,0]hept-3-yl bzw. 1,5-Dimethyl-3-aza-bicyclo[3,2,0]hept-3-yl, 3-Aza-bicyclo[3,1,1]hept-3-yl, 1,5-Dimethyl-3-aza-bicyclo[3,1,1]hept-3-yl, 7-Aza-bicyclo[2,2,1]hept-7-yl, 2-Aza-bicyclo[3,2,1]oct-2-yl, 3-Aza-bicyclo[3,2,1]oct-3-yl, 1,3,3-Trimethyl-6-aza-bicyclo[3,2,1]oct- 6-yl, 8-Aza-bicyclo[3,2,1]oct-8-yl, 3-Aza-bicyclo[3,2,2]non-3-yl oder 3-Aza-bicyclo[4,2,2]dec-3-yl, bedeutet und alk für $C_2$-$C_7$-, wie $C_2$-$C_4$-Alkylen steht, dessen freie Valenzen von benachbarten oder zueinander 1,3- oder 1,4-ständigen C-Atom ausgehen, und beispielsweise Aethylen, 1,3-Propylen oder 1,4-Butylen, bedeutet, und ihre Salze, insbesondere ihre pharmazeutisch verwendbaren Salze mit Basen.

Die Erfindung betrifft ganz besonders Verbindungen der Formel I, worin R einen gegebenenfalls durch bis und mit 3 $C_1$-$C_4$-Alkyl-, wie Methylgruppe substituierten 3-Aza-bicyclo[3,1,1]hept-3-ylrest, 6-Aza-bicyclo[3,2,1]oct-6-ylrest oder 3-Aza-bicyclo[3,2,2]non-3-ylrest, z.B. 1,5-Dimethyl-3-aza-bicyclo[3,1,1]hept-3-yl, 1,3,3-Trimethyl-6-aza-bicyclo[3,2,1]oct-6-yl oder 3-Aza-bicyclo[3,2,2]non-3-yl bedeutet und alk für $C_2$-$C_4$-Alkylen der Formel $-(CH_2)$ , worin n 2, 3 oder 4 darstellt, insbesondere für Aethylen, steht und ihre Salze, insbesondere ihre pharmazeutisch verwendbaren Salze mit Basen.

Die Erfindung betrifft in allererster Linie Verbindung der Formel I, worin R 3-Aza-bicyclo[3,1,1]hept-3-yl, 1,5-Dimethyl-3-aza-bicyclo[3,1,1]hept-3-yl, 3-Aza-bicyclo[3,2,1]oct-3-yl, 3-Aza-bicyclo[3,2,2]-non-3-yl oder 3-Aza-bicyclo[4,2,2]dec-3-yl, bedeutet und alk für $C_2$-$C_4$ Alkylen der Formel $-(CH_2)_n-$ steht, wobei n 2, 3 oder 4 bedeutet, und alk somit beispielsweise Aethylen, 1,3-Propylen oder 1,4-Butylen darstellt, und ihre Salze, insbesondere ihre pharmazeutisch verwendbaren Salze mit Basen.

Die Erfindung betrifft namentlich die in den Beispielen genannten Verbindungen der Formel I und ihre Salze, insbesondere ihre inneren Salze und pharmazeutisch verwendbaren Salze mit Basen.

Die Erfindung betrifft ferner ein auf an sich bekannten Methoden beruhendes Verfahren zur Herstellung von Verbindungen der Formel I und ihrer Salze. Dieses ist dadurch gekennzeichnet, dass man

a) in einer Verbindung der Formel

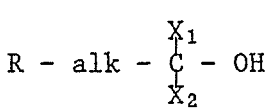 (II),

worin $X_1$ eine funktionell abgewandelte Phosphonogruppe X und $X_2$ eine freie oder funktionell abgewandelte Phosphonogruppe X bedeutet, die Gruppe(n) X in freies überführt oder

b) eine Verbindung der Formel

R - alk - $X_3$ (III),

worin $X_3$ Carboxy, Carbamyl oder Cyano bedeutet, mit phosphoriger Säure und Phosphortrichlorid umsetzt, das Primärprodukt hydrolysiert und in einem ausgehend von Verbindungen der Formel III, worin $X_3$ Carbamyl oder Cyano ist, erhaltenen Zwischenprodukt der Formel

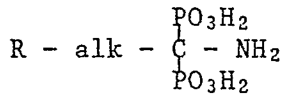 (IV)

bzw. einen Salz davon die Aminogruppe durch Behandlung mit salpetriger Säure durch Hydroxy ersetzt und gewünschtenfalls eine erhaltene Verbindung in eine andere Verbindung der Formel I und/oder eine erhaltene freie Verbindung in ein Salz oder ein erhaltenes Salz in die freie Verbindung oder in ein anderes Salz überführt.

Gemäss der Verfahrensvariante a) in Phosphono zu überführende funktionell abgewandelte Phosphonogruppen liegen beispielsweise in einer Esterform, insbesondere einer Diesterform der Formel $-P(=O)(OR_1)_2$ (VII) vor, worin $OR_1$ neben Niederalkoxy auch eine gegebenenfalls durch Niederalkyl, Niederalkoxy, Halogen, Trifluormethyl und/oder Hydroxy substituierte Phenyloxygruppe bedeuten kann.

Die Ueberführung einer funktionell abgewandelten in die freie Phosphonogruppe erfolgt in üblicher Weise durch Hydrolyse, beispielsweise in Gegenwart einer Mineralsäure, wie Salz- oder Schwefelsäure, oder durch Umsetzung mit Triniederalkyl-halogensilan, z.B. mit Trimethyl-chlorsilan oder insbesondere Trimethyl-bromsilan, vorzugsweise unter Kühlen, z.B. im Temperaturbereich von etwa 0° bis etwa 25°C.

Die Ausgangsstoffe der Formel II können beispielsweise hergestellt werden, indem man eine Verbindung der Formel

R - alk -COOH (IIa)

oder vorzugsweise das Anhydrid oder Säurechlorid derselben mit einem entsprechenden Phosphorigsäuretriester der Formel $P(OR)_3$ (IIb) in Gegenwart eines Triniederalkylamins, z.B. Triethylamin, zu einer Verbindung der Formel

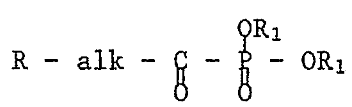 (IIc)

und diese mit einem Phosphorigsäurediester der Formel $H-P(=O)(OR)_2$ bzw. $P(OH)(OR)_2$ in Gegenwart eines Diniederalkylamins, z.B. Diethylamin, oder eines Alkalimetallniederalkanolats, z.B. Natriummethylat, zur entsprechenden Verbindung der Formel

$$\begin{array}{rcl} & & OR_1 \\ & & | \\ O = & P & - OR_1 \\ & | & \\ R - alk - & C & - OH \\ & | & \\ O = & P & - OR_1 \\ & | & \\ & & OR_1 \end{array} \qquad (II')$$

weiter umsetzt. Dabei arbeitet man jeweils vorteilhaft in Gegenwart einer Base.

Ausgangsstoffe der Formel IIa können, soweit sie nicht bekannt sind, beispielsweise hergestellt werden, indem man eine entsprechende Verbindung der Formel

Y -alk - COOR (IId),

worin Y Halogen, wie Brom ist, oder zur Herstellung von Verbindungen der Formel IIa, worin alk $C_2$-$C_7$-Alkylen, dessen freie Valenzen von benachbarten C-Atomen ausgehen, z.B. Aethylen, bedeutet, eine Verbindung der Formel

alk′ -COOR (IIe)

worin alk′ einen $C_2$-$C_7$-Alk-1-enylrest bedeutet, mit einem Aza-bicyclo alkan der Formel R-H (IIf) umsetzt, jeweils den erhaltenen Ester zur Säure hydrolysiert und diese, z.B. mittels Phosphorpentachlorid, anhydridisiert.

Neue Ausgangsstoffe IIf können ihrerseits durch Reduktion entsprechender (Oxo- bzw. Dioxo)azabicycloalkane, insbesondere solche, in denen sich die Oxogruppe(n) in $\alpha$-Stellung zum N-Atom befindet (befinden), hergestellt werden. Als Reduktionsmittel für diesen Zweck ist insbesondere Natriumdihydro-bis(2-methoxyäthoxy)-aluminat geeignet. So erhält man 3-Aza-bicyclo[3,1,1]heptan, 1,5-Dimethyl-3-aza-bicyclo[3,1,1]heptan, 3-Aza-bicyclo[3,2,0]heptan und 1,5-Dimethyl-3-aza-bicyclo[3,2,0]heptan elegant durch Behandlung von 2,4-Dioxo-3-aza-bicyclo[3,1,1]heptan, 1,5-Dimethyl-2,4-dioxo-3-aza-bicyclo[3,1,1]heptan, 2,4-Dioxo-3-aza-bicyclo[3,2,0]heptan bzw. 1,5-Dimethyl-2,4-dioxo-3-aza-bicyclo[3,2,0]heptan mit Natriumdihydrobis-(2-methoxyäthoxy)-aluminat (etwa 70%ig in Toluol) bei etwa 20-40°C in Toluol als Lösungsmittel und hydrolytische Aufarbeitung in Gegenwart von Natriumhydroxid.

Die Umsetzung von Verbindungen der Formel III mit phosphoriger Säure und Phosphortrichlorid gemäss der Verfahrensvariante b) erfolgt in üblicher Weise, wobei die Phophorigsäurekomponente vorzugsweise durch Reaktion überschüssigen Phosphortrichlorides mit wasserhaltiger Phosphorsäure, z.B. mit handelsüblicher etwa 75%-iger bis etwa 95%-iger, vorzugsweise etwa 85%-iger Phosphorsäure, in situ gebildet wird. Die Umsetzung wird vorteilhaft unter Erwärmen, z.B. auf etwa 70° bis etwa 120°C, in einem geeigneten Lösungsmittel, wie Tetrachloräthan, Trichloräthan, Chlorbenzol, Chlortoluol oder Paraffinöl, und unter hydrolytischer Aufarbeitung durchgeführt.

Die Behandlung von Zwischenprodukten der Formel IV mit salpetriger Säure erfolgt in üblicher Weise unter Freisetzung derselben in wässriger Lösung aus einem ihrer Salze, z.B. aus Natriumnitrat, durch Säurebehandlung, z.B. Einwirkung von Salzsäure, wobei intermediär ein entsprechendes, instabiles Diazoniumsalz, z.B. -chlorid, gebildet wird, das unter Einführung der $\alpha$-Hydroxygruppe Stickstoff abspaltet.

Die Ausgangsstoffe der Formel III können, soweit sie nicht bekannt sind, beispielsweise hergestellt werden, indem man eine entsprechende Verbindung der Formel

Y - alk - COOR (IId),

worin Y Halogen, wie Brom ist, oder zur Herstellung von Verbindungen der Formel IIIa, worin alk $C_2$-$C_7$-Alkylen, dessen freie Valenzen von benachbarten C-Atomen ausgehen, z.B. Aethylen bedeutet, eine Verbindung der Formel
alk′ - COOR (IIe)

worin alk′ einen $C_2$-$C_7$-Alk-1-enylenrest bedeutet, mit einem Aza-bicyclo alkan der Formel R-H (IIf) umsetzt und jeweils den erhaltenen Ester zur Säure hydrolysiert.

Verfahrensgemäss oder nach einem anderen an sich bekannten Verfahren erhaltene Verbindungen der Formel I können in an sich bekannter Weise in andere Verbindungen der Formel I überführt werden.

Die neuen Verbindungen können, je nach der Wahl der Ausgangsstoffe und Arbeitsweisen, in Form eines der möglichen Isomeren oder als Gemisch derselben, z.B. je nach der Anzahl der asymmetrischen Kohlenstoffatome als reine optische Isomere, wie Antipoden oder als Isomerengemische, wie Racemate, Diastereoisomerengemische oder Racematgemische, vorliegen.

Erhaltene Diastereomerengemische und Racematgemische können auf Grund der physikalisch-chemischen Unterschiede der Bestandteile in bekannter Weise in die reinen Isomeren, Diastereomeren oder Racemate aufgetrennt werden, beispielsweise durch Chromatographie und/oder fraktionierte Kristallisation.

Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen, oder durch Umsetzung eines sauren Endstoffes mit einer mit der racemischen Säure Salze bildenden optisch aktiven Base und Trennung der auf diese Weise erhaltenen Salze, z.B. auf Grund ihrer verschiedenen Löslichkeiten, in die Diastereomeren, aus denen die Antipoden durch Einwirkung geeigneter Mittel freigesetzt werden können, zerlegen. Vorteilhaft isoliert man den wirksameren der beiden Antipoden.

Erhaltene freie Verbindungen der Formel I können durch partielle oder vollständige Neutralisation mit einer der eingangs genannten Basen in Basesalze überführt werden.

Erhaltene Salze können in an sich bekannter Weise in die freien Verbindungen umgewandelt werden, z.B. durch Behandeln mit einem sauren

Reagens, wie einer Mineralsäure, bzw. einer Base, z.B. Alkalilauge.

Die Verbindungen einschliesslich ihrer Salze können auch in Form ihrer Hydrate erhalten werden oder das zur Krisallisation verwendete Lösungsmittel einschliessen.

Infolge der engen Beziehung zwischen den neuen Verbindungen in freier Form und in Form ihrer Salze sind im vorausgegangenen und nachfolgend unter den freien Verbindungen oder ihren Salzen sinn-und zweckgemäss gegebenenfalls auch die entsprechenden Salze bzw. freien Verbindungen zu verstehen.

Die Erfindung betrifft auch diejenigen Ausführungsformen des Verfahrens, nach denen man von einer auf irgendeiner Stufe des Verfahrens als Zwischenprodukt erhältlichen Verbindungen ausgeht und die fehlenden Schritte durchführt oder einen Ausgangsstoff in Form eines Salzes und/oder Racemates bzw. Antipoden verwendet oder insbesondere unter den Reaktionsbedingungen bildet.

Beim Verfahren der vorliegenden Erfindung werden vorzugsweise solche Ausgangsstoffe verwendet, welche zu den eingangs als besonders wertvoll geschilderten Verbindungen führen. Neue Ausgangsstoffe und Verfahren zu deren Herstellung bilden ebenfalls einen Gegenstand der Erfindung.

Bei den erfindungsgemässen pharmazeutischen Präparaten, welche Verbindungen der Formel I oder pharmazeutisch verwendbare Salze davon enthalten, handelt es sich um solche zur enteralen, wie oralen oder rektalen, und parenteralen Verabreichung, welche den pharmakologischen Wirkstoff allein oder zusammen mit einem pharmazeutisch anwendbaren Trägermaterial enthalten. Die Dosierung des Wirkstoffs hängt von der Warmblüter-Spezies, dem Alter und dem individuellen Zustand, sowie von der Applikationsweise ab. Im Normalfall ist für einen etwa 75 kg schweren Warmblüter bei oraler Applikation eine ungefähre Tagesdosis von etwa 30 bis 1000 mg, vorzugsweise etwa 100 bis 1000 mg, bzw. bei intravenöser Verabreichung etwa 1 bis 50, vorzugsweise 5 bis 10 mg, vorteilhaft in mehreren gleichen Teildosen verteilt, zu veranschlagen.

Die neuen pharmazeutischen Präparate enthalten z.B. von etwa 10 % bis etwa 80 %, vorzugseise von etwa 20 % bis etwa 60 % des Wirkstoffs. Erfindungsgemässe pharmazeutische Präparate zur enteralen bzw. parenteralen Verabreichung sind z.B. solche in Dosiseinheitsformen wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragéee-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. vom Mais-, Weizen-, Reis- oder Kartoffelstärke, Gelatine, Tragaganth, Methylcellulose und/oder Polyvinylpyrrolidon, und/oder, wenn erwünscht, Sprengmittel, wie die obgenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium-oder Calciumstearat, und/oder Polyethylenglykol. Dragéee-Kerne werden mit geeigneten, gegebenenfalls Magensaft-resistenten Ueberzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyethyleglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffin kohlenwasserstoffe, Polyethylenglykole oder höhere Alkanole. Ferner können auch Gelatine-Rektalkapseln verwendet werden, die eine Kombination des Wirkstoffs mit einer Grundmasse enthalten; als Grundmassenstoffe kommen z.B. flüssige Triglyceride, Polyethylenglykole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Ethyloleat oder Triglyceride, verwendet, oder wässrige Injektionssuspensionen, welche viskositätserhöhende Stoffe, z.B. Natriumcarboxymethylcellulose, Sorbit und/oder Dextran und gegebenenfalls auch Stabilisatoren enthalten.

Die vorliegende Erfindung betrifft ebenfalls die

Verwendung der Verbindungen der Formeln I und ihrer Salze, vorzusweise zur Behandlung von Entzündungen, in erster Linie von entzündlichen Erkrankungen des rheumatischen Formenkreises, besonders der chronischen Arthritis, ebenso zur Behandlung von auf Störungen des Calciumstoffwechsels zurückzuführenden Ekrankungen, z.B. von Osteoporeosen.

Dosierungen unter 0,001 mg/kg Körpergewicht beeinflussen die pathologische Verkalkung bzw. die Auflösung von harten Geweben nur unerheblich. Bei Dosierungen von über 100 mg/kg Körpergewicht können langfristig toxische Nebenwirkungen auftreten. Die Verbindungen der Formel I und ihre Salze können sowohl oral als auch hypertonischer Lösung subkutan, intramuskulär oder intravenös appliziert werden. Die bevorzugten Tages dosen für diese Anwendungen liegen bei oraler Anwendung im Bereich von etwa 0,1 bis 5 mg/kg, bei subkutaner und intramuskulärer Applikation im Bereich von etwa 0,1 bis 1 mg/kg und bei intravenöser Applikation im Bereich von etwa 0,01 bis 2 mg/kg.

Die Dosierung der verwendeten Verbindungen ist jedoch variabel und hängt von den jeweiligen Konditionen wie Art und Schwere der Erkrankung, Dauer der Behandlung und der jeweiligen Verbindung ab. Einzeldosen enthalten beispielsweise von 0,01 bis 10 mg, Dosiseinheitsformen für parenterale, wie intravenöse Applikation z.B. von 0,01 bis 0,1 mg, vorzugsweise 0,02 bis 0,08 mg, orale Dosiseinheitsformen z.B. von 0,2 bis 2,5 mg, vorzugsweise 0,3 bis 1,5 mg pro kg Körpergewicht. Die bevorzugte Einzeldosierung beträgt bei oraler Applikation 10 bis 100 mg und bei intravenöser Applikation 0,5 bis 5 mg. Es können jedoch bis zu 4 Einzeldosen täglich verabreicht werden. Die höheren Dosierungen bei oraler Applikation sind infolge der begrenzten Resorption erforderlich. Bei längerdauernden Behandlungen kann nach anfänglich höherer Dosierung normalerweise auf niedrige Dosierungen umgestellt werden, um den gewünschten Effekt aufrechtzuerhalten.

Die nachfolgenden Beispiele illustrieren die oben beschriebene Erfindung; sie sollen jedoch diese in ihrem Umfang in keiner Weise einschränken. Temperaturen sind in Celsiusgraden angegeben.

Beispiel 1:

11,68 g (0,05 Mol) 3-(1,5-Dimethyl-3-aza-bicyclo[3,1,1]hept-3-yl)-propionsäure-hydrochlorid, 6,6 ml 84%ige Phosphorsäure und 23 ml Chlorbenzol werden unter Rühren auf 105° erhitzt. 13,5 ml Phosphortrichlorid werden langsam zugetropft, anschliessend hält man das Reaktionsgemisch noch 3 Stunden bei 105°. Dann wird unter vermindertem Druck das Chlorbenzol abdestilliert. Die verbleibende zähe Masse wird mit 50 ml Wasser versetzt und unter Rühren und Rückfluss 1 Stunde lang zum Sieden erhitzt. Anschliessend wird unter vermindertem Druck völlig eingedampft. Beim Verrühren des Rückstands mit Aceton erhält man nach Abdekantieren ein farbloses Oel, welches in 50 ml heissem Wasser gelöst wird. Auf Zusatz von 140 ml heissem Methanol enthält man nach dem Abkühlen farblose Kristalle der 3-(1,5-Dimethyl-3-aza-bicyclo[3,1,1]hept-3-yl)-1-hydroxy-propan-1,1-diphosphonsäure, Smp. 245-247° (Zers.); Formel

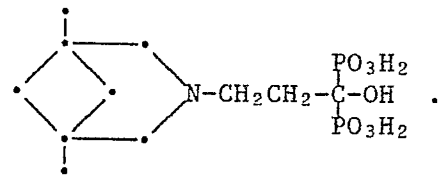

Das als Ausgangsmaterial dienende 3-(1,5-Dimethyl-3-aza-bicyclo[3,1,1]hept-3-yl)-propionsäure-hydrochlorid kann folgendermassen hergestellt werden:

Zu einer gerührten, auf 0-5° C gekühlten Lösung von 71 g N-tert.Butylmethacrylamid, 50,8 g Triethylamin und 570 ml Methylenchlorid wird eine Lösung von 52,7 g Methacrylsäurechlorid in 570 ml Methylenchlorid getropft. Nach der Zugabe wird 2 1/2 Stunden gerührt und die Reaktionslösung 4 Tage stehen gelassen. Das eingeengte Reaktionsgut wird in 0,5 l Diethylether aufgenommen, filtriert und die Lösung eingeengt. Das erhaltene rote Oel wird mit Hexan-Diethylether (4:1) an Kieselgel filtriert und man erhält so 3-(1,5-Dimethyl-3-aza-bicyclo[3,1,1]hept-3-yl)-propionsäure-hydrochlorid als weisses, kristallines Produkt (Smp. 47-48° C).

Eine Lösung von 52,5 g 2,6-Dimethyl-4-tert.butyl-1,6-heptadien3,5-dion und 0,5 g 2,6-Di-tert.butyl-p-kresol in 3,9 l Methylenchlorid wird während 30 Stunden mit ultraviolettem Licht bestrahlt. Nach dem Einengen wird das Rohprodukt mit Toluol-Diethylether (15:1) an 3 kg Kieselgel 60 chromatographiert. Man erhält die Titelverbindung als weisse Kristalle mit einem Smp. von 65-65,5° C (Umkristallisation aus n-Pentan bei -70° C).

Die Lösung von 47,6 g 1,5-Dimethyl-3-tert.butyl-3-aza-bicyclo[3.1.1]heptan-2,4-dion in 215 ml Trifluoressigsäure wird während 6 Stunden am Rückfluss erwärmt. Die eingedampfte Reaktionsmischung wird in Diethylether aufgenommen, und das kristallin ausgefallene Produkt wird filtriert und mit Diethylether gewaschen. Man erhält so die Titelverbindung mit einem Smp. von 195-196° C.

Zur gerührten Suspension von 1,5-Dimethyl-3-aza-bicyclo[3.1.1]heptan-2,4-dion werden unter Stickstoffatmosphäre 80 ml Natriumdihydro-bis-(2-methoxyethoxy)-aluminat-Toluollösung (70 %; FLUKA) zugetropft. Während der Zugabe wird die Temperatur durch externe Kühlung im Eisbad im Bereich von 25-35° C gehalten. Nach beendeter Zugabe wird 15 Minuten bei Raumtemperatur (RT) nachgerührt und anschliessend 1 Stunde unter Rückfluss erwärmt. Nach dem Abkühlen im Eisbad werden bei 10-15° C 25,5 ml konzentrierte Natronlauge zugetropft. Die organische Phase wird abdekantiert, und die Wasserphase mit Toluol gewaschen. Die vereinigten organischen Phasen werden zweimal mit 100 ml Wasser und einmal mit 70 ml Sole gewaschen. Nach der Zugabe von Magnesiumsulfat wird die organische Phase filtriert und am Wasserstrahlvakuum eingeengt. Das bräunliche Oel wird in 50 ml absolutem Diethylether gelöst. Die Titelverbindung wird durch Einleiten von HCl-Gas als kristallines Produkt erhalten, welches nach dem Abnutschen nochmals in Diethylether aufgeschlämmt und wiederum abgenutscht und zuletzt am Hochvakuum über Nacht getrocknet wird. Man erhält 1,5-Dimethyl-3-aza-bicyclo[3.1.1]heptan-hydrochlorid als farblose Kristalle mit einem Smp. von 144,5-145,5°.

18,8 g 1,1'-Dimethyl-3-aza-bicyclo[3,1,1]heptan (0,15 Mol) werden in 50 ml Diäthyläther vorgelegt und unter Rühren allmählich mit 15,1 g Acrylsäureäthylester versetzt. Es bildet sich unter leichtem Anstieg der Temperatur eine klare Lösung. Nach Stehen über Nacht bei Raumtemperatur wird der Aether abdestilliert. Das zurückbleibende Oel stellt den rohen 3-(1,1'-Dimethyl-3-aza-bicyclo[3,1,1]hept-3-yl)-propionsäureäthylester dar.

32.06 g 3-(1,1'-Dimethyl-3-aza-bicyclo[3,1,1]hept-3-yl)-propionsäureäthylester werden mit 600 ml 4-n Chlorwasserstoffsäure 24 Stunden zum Rückfluss erhitzt. Dann wird unter vermindertem Druck völlig eingedampft und der kristalline Rückstand mit Aceton verrieben. Nach Abnutschen, Waschen und Trocknen der Kristalle erhält man das 3-(1,1'-Dimethyl-3-aza-bicyclo[3,1,1]hept-3-yl)-propionsäure-hydrochlorid Smp. 226-228°C (Zers.).

Beispiel 2:

Eine wässrige Lösung von 5,15 g (15 mMol) 3-(1,5-Dimethyl-3-aza-bicyclo[3,1,1]hept-3-yl)-1-hydroxy-propan-1,1-diphosphonsäure und 7,26 g Tris-(hydroxymethyl)-aminomethan wird unter vermindertem Druck zur Trockne eingedampft. Der Rückstand wird in Methanol gelöst und anschliessend mit 2-Propanol versetzt, worauf sich farblose Kristalle von Di-[tris(hydroxymethyl)methyl]ammonium-(1,5-dimethyl-3-azabicyclo-[3,1,1]hept-3-yl)-1-hydroxy-propan-1,1-diphosphonat, d.h. des partiellen Salzes von 3-(1,5-Dimethyl-3-aza-bicyclo[3,1,1]hept-3-yl)-1-hydroxy-propan-1,1-diphosphonsäure mit 3-Hydroxy-2,2-bis(hydroxymethyl)-1-aminopropan, Smp. 171-174° (Zers.), abscheiden.

Beispiel 3:

Analog Beispiel 1 erhält man aus 3-Aza-bicyclo[3,2,2]nonan über 3-(3-Aza-bicyclo[3,2,2]non-3-yl)-propionsäure-hydrochlorid, Smp. 227-229 die 3-(3-Aza-bicyclo[3,2,2]non-3-yl)-1-hydroxy-propan-1,1-diphosphonsäure, Smp. 145-147°; Formel

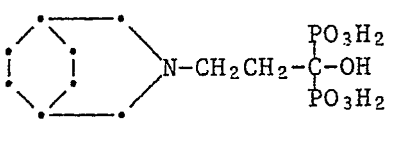

Beispiel 4:

15,3 g 1,3,3-Trimethyl-6-aza-bicyclo[3,2,1]octan (0,15 Mol) werden in 50 ml Diäthyläther vorgelegt und unter Rühren allmählich mit 15,1 g Acrylsäureäthylester versetzt. Es bildet sich unter leichtem Anstieg der Temperatur eine klare Lösung. Nach Stehen über Nacht bei Raumtemperatur wird der Aether abdestilliert. Das zurückbleibende Oel wird vakuumdestilliert. Man erhält den 3-(1,3,3-Trimethyl-6-aza-bicyclo[3,2,1)oct-6-yl)-propionsäureäthylester, Kp. 77°/0,013 mbar.

16,8 g 3-(1,3,3-Trimethyl-6-aza-bicyclo[3,2,1]oct-6-yl)-propionsäureäthylester (0,066 Mol) werden mit 300 ml 4-n Chlorwasserstoffsäure 24 Stunden zum Rückfluss erhitzt. Dann wird unter vermindertem Druck völlig eingedampft und der kristalline Rückstand mit Aceton verrieben. Nach Abnutschen, Waschen und Troknen der Kristalle erhält man das 3-(1,3,3-Trimethyl-6-aza-bicyclo[3,2,1]oct-6-yl)-propionsäure-hydrochlorid Smp. 183-185°C (Zers.).

Analog Beispiel 1 erhält man aus 3-(1,3,3-Trimethyl-6-aza-bicyclo[3,2,1]-oct-6-yl)-propionsäure-hydrochlorid die 3-(1,3,3-Trimethyl-6-aza-bicyclo[3,2,1]oct-6-yl)-1-hydroxypropan-1,1-diphosphonsäure, Smp. 195-198°C (Zers.); Formel

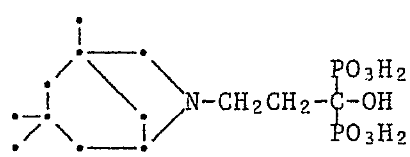

Beispiel 5:

In analoger Weise wie in Beispiel 1 beschrieben kann man ferner herstellen:

3-(3-Aza-bicyclo[3,1,0]hex-3-yl)-1-hydroxy-propan-1,1-diphosphonsäure;
2-(1,5-Dimethyl-3-aza-bicyclo[3,1,0]hex-3-yl)-1-hydroxy-propan-1,1-diphosphonsäure;
3-(3-Aza-bicyclo[3,2,0]hept-3-yl)-1-hydroxy-propan-1,1-diphosphonsäure;
3-(1,5-Dimethyl-3-aza-bicyclo[3,2,0]hept-3-yl)-1-hydroxy-propan-1,1-diphosphonsäure;
3-(3-Aza-bicyclo[3,1,1]hept-3-yl)-1-hydroxy-propan-1,1-diphosphonsäure;
3-(7-Aza-bicyclo[2,2,1]hept-7-yl)-1-hydroxy-propan-1,1-diphosphonsäure,
3-(2-Aza-bicyclo[3,2,1]oct-2-yl)-1-hydroxy-propan-1,1-diphosphonsäure;
3-(3-Aza-bicyclo[3,2,1]oct-3-yl)-1-hydroxy-propan-1,1-diphosphonsäure;
3-(8-Aza-bicyclo[3,2,1]oct-8-yl)-1-hydroxy-propan-1,1-diphosphonsäure und
3-(3-Aza-bicyclo[4,2,2]dec-3-yl)-1-hydroxy-propan-1,1-diphosphonsäure
und ihre Salze, z.B. ihre Di- oder Tetranatriumsalze.

Beispiel 6:

Tabletten, enthaltend 25 mg Wirkstoff, z.B. 3-(1,5-Dimethyl-3-aza-bicyclo[3,1,1]hept--3-yl)-1-hydroxy-propan-1,1-diphosphonsäure oder ein Salz, z.B. das Dinatriumsalz, davon, können folgendermassen hergestellt werden:

Bestandteile (für 1000 Tabletten)

| | | |
|---|---|---|
| Wirkstoff | | 25,0 g |
| Lactose | | 100,7 g |
| Weizenstärke | | 7,5 g |
| Polyäthylenglykol 6000 | | 5,0 g |
| Talkum | | 5,0 g |
| Magnesiumstearat | | 1,8 g |
| entmineralisiertes Wasser | q.s. | |

Herstellung:
Sämtliche festen Ingredienzien werden zunächst durch ein Sieb mit 0,6 mm Maschenweite getrieben. Dann wird der Wirkstoff, die Lactose, das Talkum, das Magnesiumstearat und die Hälfte der Stärke vermischt. Die andere Hälfte der Stärke wird in 40 ml Wasser suspendiert und diese Suspension zu einer siedenden Lösung des Polyäthylenglykols in 100 ml Wasser hinzugegeben und das Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Durchmesser verpresst.

Beispiel 7:
Kautabletten, enthaltend 30 mg Wirkstoff, z.B. 3-(1,5-Dimethyl-3-aza-bicyclo[3,1,1]hept--3-yl)-1-hydroxy-propan-1,1-diphosphonsäure oder ein Salz, z.B. das Dinatriumsalz davon, können z.B. folgendermassen hergestellt werden:

Zusammensetzung: (für 1000 Tabletten)

| | | |
|---|---|---|
| Wirkstoff | | 30,0 g |
| Mannit | | 267,0 g |
| Lactose | | 179,5 g |
| Talkum | | 20,0 g |
| Glycin | | 12,5 g |
| Stearinsäure | | 10,0 g |
| Saccharin | | 1,0 g |
| 5 %-ige Gelatinelösung | q.s. | |

Herstellung:
Alle festen Ingredienzien werden zunächst durch ein Sieb mit 0,25 mm Maschenweite getrieben. Der Mannit und die Lactose werden gemischt, unter Hinzufügen von Gelatinelösung granuliert, durch ein Sieb mit 2 mm Maschenweite getrieben, bei 50° getrocknet und nochmals durch ein Sieb mit 1,7 mm Maschenweite getrieben. Der Wirkstoff, das Glycin und das Saccharin werden sorgfältig vermischt, der Mannit, das Lactosegranulat, die Stearinsäure und das Talkum hinzugegeben, das Ganze gründlich vermischt und zu beidseitig konkaven Tabletten von etwa 10 mm Durchmesser mit Bruchrille auf der Oberseite verpresst.

Beispiel 8:
Tabletten, enthaltend 100 mg Wirkstoff, z.B. 3-(1,5-Dimethyl-3-aza-bicyclo[3,1,1]hept--3-yl)-1-hydroxy-propan-1,1-diphosphonsäure oder ein Salz, z.B. das Dinatriumsalz davon, können folgendermassen hergestellt werden:

Zusammensetzung (für 1000 Tabletten)

| | | |
|---|---|---|
| Wirkstoff | | 100,o g |
| Lactose | | 248,5 g |
| Maisstärke | | 17,5 g |
| Polyäthylenglykol 6000 | | 5,0 g |
| Talkum | | 15,0 g |
| Magnesiumstearat | | 4,0 g |
| entmineralisiertes Wasser | q.s. | |

Herstellung:
Die festen Ingredienzien werden zunächst durch ein Sieb mit 0,6 mm Maschenweite getrieben. Dann werden Wirkstoff, Lactose, Talkum, Magnesiumstearat und die Hälfte der Stärke innig vermischt. Die andere Hälfte der Stärke wird in 65 ml Wasser suspendiert und diese Suspension zu einer siedenden Lösung des Polyäthylenglykols in 260 ml Wasser hinzugegeben. Der erhaltene Kleister wird zu den pulverförmigen Substanzen hinzugefügt, das Ganze vermischt und granuliert, erforderlichenfalls unter Zugabe von Wasser. Das Granulat wird über Nacht bei 35° getrocknet, durch ein Sieb mit 1,2 mm Maschenweite getrieben und zu beidseitig konkaven Tabletten von etwa 10 mm Durchmesser mit Bruchkerbe auf der Oberseite verpresst.

Beispiel 9:
In analoger Weise wie in den Beispielen 6-8 beschrieben kann man auch pharmazeutische Präparate, enthaltend eine andere Verbindung gemäss einem der Beispiele 1-5 herstellen.

**Patentansprüche**

1. Neue Azacycloalkylalkandiphosphonsäuren der Formel

$$R - alk - \underset{PO_3H_2}{\overset{PO_3H_2}{\underset{|}{\overset{|}{C}}}} - OH \qquad (I)$$

worin R einen über das Azaringglied gebundene, aus 3- bis und mit 8-gliedrigen Ringsystemen aufgebaute Aza-bicycloalkylrest bedeutet und alk Niederalkylen darstellt, und ihre Salze.

2. Verbindungen gemäss Anspruch 1 der Formel I, worin R einen über das Azaringglied gebundenen, aus 3- bis und mit 8-gliedrigen Ringsystemen aufgebauten Aza-bicyclo-

$C_6$-$C_{10}$-alkylrest bedeutet und alk $C_1$-$C_7$-Alkylen darstellt, und ihre Salze.

3. Verbindungen gemäss Anspruch 1 der Formel I, worin R einen über das Azaringglied gebundenen, aus 4- bis 7-gliedrigen Ringsystemen aufgebauten Aza-bicyclo -$C_6$-$C_{10}$-alkylrest bedeutet und alk für $C_2$-$C_7$-Alkylen steht, dessen freie Valenzen von benachbarten oder zueinander 1,3- oder 1,4-ständigen C-Atom ausgehen, und ihre Salze.

4. Verbindungen gemäss Anspruch 1 der Formel I, worin R einen gegebenenfalls durch bis und mit 3 $C_1$-$C_4$-Alkyl-, wie Methylgruppen substituierten 3-Aza-bicyclo[3,1,1]hept-3-ylrest, 6-Aza-bicyclo[3,2,1]oct-6-ylrest oder 3-Aza-bicyclo[3,2,2]non-3-ylrest bedeutet und alk für $C_2$-$C_4$-Alkylen der Formel -$(CH_2)_n$ steht, wobei n 2, 3 oer 4 darstellt, und ihre Salze.

5. Verbindungen gemäss Anspruch 1 der Formel I, worin R 3-Aza-bicyclo[3,1,1]hept-3-yl, 1,5-Dimethyl-3-aza-bicyclo[3,1,1]hept-3-yl, 7-Aza-bicyclo[2,2,1]hept-7-yl, 2-Aza-bicyclo[3,2,1]oct-2-yl, 3-Aza-bicyclo[3,2,1]oct-3-yl, 1,3,3-Trimethyl-6-aza-bicyclo[3,2,1]oct-6-yl, 8-Aza-bicyclo[3,2,1]oct-8-yl, 3-Aza-bicyclo[3,2,2]non-3-yl oder 3-Aza-bicyclo[4,2,2]dec-3-yl bedeutet und alk für $C_2$-$C_4$-Alkylen der Formel -$CH_2)_n$-steht, wobei n 2, 3 oder 4 bedeutet, und ihre Salze.

6. Verbindungen gemäss Anspruch 1 der Formel I, worin R 3-Aza-bicyclo[3,1,1]hept-3-yl, 1,5-Dimethyl-3-aza-bicyclo[3,1,1]hept-3-yl, 7-Aza-bicyclo[2,2,1]hept-7-yl, 2-Aza-bicyclo[3,2,1]oct-2-yl, 3-Aza-bicyclo[3,2,1]oct-3-yl, 8-Aza-bicyclo[3,2,1]oct-8-yl, 3-Aza-bicyclo[3,2,2]non-3-yl oder 3-Aza-bicyclo [4,2,2]dec-3-yl bedeutet und alk für $C_2$-$C_4$-Alkylen der Formel -$CH_2)_n$- steht, wobei n 2, 3 oder 4 bedeutet, und ihre Salze.

7. 3-(1,5-Dimethyl-3-aza-bicyclo[3,1,1]hept-3-yl)-1-hydroxy-propan-1,1-diphosphonsäure oder ein Salz davon.

8. 3-(3-Aza-bicyclo[3,2,2]non-3-yl)-1-hydroxy-propan-1,1-diphosphonsäure oder ein Salz davon.

9. 3-(1,3,3-Trimethyl-6-aza-bicyclo[3,2,1]oct-6-yl)1-hydroxy-propan-1,1-diphosphonsäure oder ein Salz davon.

10. Eine Verbindung gemäss einem der Anspruche 1 bis 3 und 5 bis 8 zur Anwendung in einem Verfahren zur therapeutischen Behandlung des menschlichen oder tierischen Körpers.

11. Eine Verbindung gemäss einem der Ansprüche 4 und 9 zur Anwendung in einem Verfahren zur therapeutischen Behandlung des menschlichen oder tierischen Körpers.

12. Pharmazeutische Präparate enthaltend eine Verbindung gemäss einem der Ansprüche 1 bis 3, 5 bis 8 und 10 neben üblichen pharmazeutischen Hilfsstoffen.

13. Pharmazeutische Präparate enthaltend eine Verbindung gemäss einem der Ansprüche 4 und 9 neben üblichen pharmazeutischen Hilfsstoffen.

14. Verfahren zur Herstellung neuer Azacycloalkylalkandiphosphonsäuren der Formel

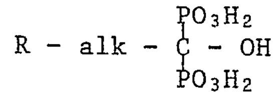

(I)

worin R einen über das Azaringglied gebundenen, aus 3- bis und mit 8-gliedrigen Ringsystemen aufgebaute Aza-bicycloalkylrest bedeutet und alk Niederalkylen darstellt, und ihrer Salze, dadurch gekennzeichnet, dass man

a) in einer Verbindung der Formel

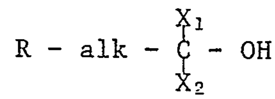

(II),

worin $X_1$ eine funktionell abgewandelte Phosphonogruppe X und $X_2$ eine freie oder funktionell abgewandelte Phosphonogruppe X bedeutet, die Gruppe(n) X in freies Phosphono überführt oder

b) eine Verbindung der Formel

R -alk - $X_3$ (III),

worin $X_3$ Carboxy, Carbamyl oder Cyano bedeutet, mit phosphoriger Säure und Phosphortrichlorid umsetzt, das Primärprodukt hydrolysiert und in einem ausgehend von Verbindungen der Formel III, worin $X_3$ Carbamyl oder Cyano ist, erhaltenen Zwischenprodukt der Formel

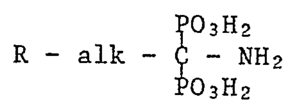

(V)

bzw. einen Salz davon die Aminogruppe durch Bedhandlung mit salpetriger Säure durch Hydroxy ersetzt und gewünschtenfalls eine erhaltene Verbindung in eine andere Verbindung der Formel I und/oder eine erhaltene freie Verbindung in ein Salz oder ein erhaltenes Salz in die freie Verbindung oder in ein anderes Salz überführt.

15. Verwendung einer Verbindung gemäss einem der Ansprüche 1 bis 9 zur Herstellung eines zur Behandlung von Calcium-Stoffwechselerkrankungen, geeigneten pharmazeutischen Präparates.

**Patentansprüche für folgende Vetragsstaaten: GR, ES**

1. Verfahren zur Herstellung neuer Azacycloalkylalkandiphosphonsäuren der Formel

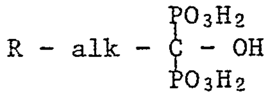 (I)

worin R einen über das Azaringglied gebundenen, aus 3- bis und mit 8-gliedrigen Ringsystemen aufgebaute Aza-bicycloalkylrest bedeutet und alk Niederalkylen darstellt, und ihrer Salze, dadurch gekennzeichnet, dass man

a) in einer Verbindung der Formel

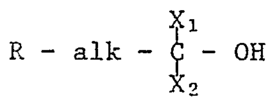 (II),

worin $X_1$ eine funktionell abgewandelte Phosphonogruppe X und $X_2$ eine freie oder funktionell abgewandelte Phosphonogruppe X bedeutet, die Gruppe(n) X in freies Phosphono überführt oder

b) eine Verbindung der Formel

R - alk - $X_3$ (III),

worin $X_3$ Carboxy, Carbamyl oder Cyano bedeutet, mit phosphoriger Säure und Phosphortrichlorid umsetzt, das Primärprodukt hydrolysiert und in einem ausgehend von Verbindungen der Formel III, worin $X_3$ Carbamyl oder Cyano ist, erhaltenen Zwischenprodukt der Formel

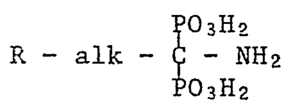 (V)

bzw. einen Salz davon die Aminogruppe durch Behandlung mit salpetriger Säure durch Hydroxy ersetzt und gewünschtenfalls eine erhaltene Verbindung in eine andere Verbindung der Formel I und/oder eine erhaltene freie Verbindung in ein Salz oder ein erhaltenes Salz in die freie Verbindung oder in ein anderes Salz überführt.

2. Verfahren gemäss Anspruch 1, dadurch gekennzeichnet, dass man von einer auf irgendeiner Stufe des Verfahrens als Zwischenprodukt erhältlichen Verbindungen ausgeht und die fehlenden Schritte durchführt oder einen Ausgangsstoff in Form eines Salzes und/oder Racemates bzw. Antipoden verwendet oder insbesondere unter den Reaktionsbedingungen bildet.

3. Verfahren gemäss Anspruch 1 oder 2 zur Herstellung von Verbindungen gemäss Anspruch 1 der Formel I, worin R einen über das Azaringglied gebundenen, aus 3- bis und mit 8-gliedrigen Ringsystemen aufgebauten Aza-bicyclo-$C_6$-$C_{10}$-alkylrest bedeutet und alk $C_1$-$C_7$-Alkylen darstellt, und ihrer Salze.

4. Verfahren gemäss Anspruch 1 oder 2 zur Herstellung von Verbindungen gemäss Anspruch 1 der Formel I, worin R einen über das Azaringglied gebundenen, aus 4- bis 7-gliedrigen Ringsystemen aufgebauten Aza-bicyclo-$C_6$-$C_{10}$-alkylrest bedeutet und alk für $C_2$-$C_7$-Alkylen steht, dessen freie Valenzen von benachbarten oder zueinander 1,3- oder 1,4-ständigen C-Atom ausgehen, und ihrer Salze.

5. Verfahren gemäss Anspruch 1 oder 2 zur Herstellung von Verbindungen gemäss Anspruch 1 der Formel I, worin R einen gegebenenfalls durch bis und mit 3 $C_1$-$C_4$-Alkyl-, wie Methylgruppen substituierten 3-Aza-bicyclo[3,1,1]hept-3-ylrest, 6-Aza-bicyclo[3,2,1]oct-6-ylrest oder 3-Aza-bicyclo[3,2,2]non-3-ylrest bedeutet und alk für $C_2$-$C_4$-Alkylen der Formel $-CH_2)_n$ steht, wobei n 2, 3 oer 4 darstellt, und ihrer Salze.

6. Verfahren gemäss Anspruch 1 oder 2 zur Herstellung von Verbindungen gemäss Anspruch 1 der Formel I, worin R 3-Aza-bicyclo[3,1,1]hept-3-yl, 1,5-Dimethyl-3-aza-bicyclo[3,1,1]hept-3-yl, 7-Aza-bicyclo[2,2,1]hept-7-yl, 2-Aza-bicyclo[3,2,1]oct-2-yl, 3-Aza-bicyclo[3,2,1]oct-3-yl, 1,3,3-Trimethyl-6-aza-bicyclo[3,2,1]oct-6-yl, 8-Aza-bicyclo[3,2,1]oct-8-yl, 3-Aza-bicyclo[3,2,2]non-3-yl oder 3-Aza-bicyclo [4,2,2]dec-3-yl bedeutet und alk für $C_2$-$C_4$-Alkylen der Formel $-CH_2)_n$-steht, wobei n 2, 3 oder 4 bedeutet, und ihrer Salze.

7. Verfahren gemäss Anspruch 1 oder 2 zur Herstellung von Verbindungen gemäss Anspruch 1 der Formel I, worin R 3-Aza-bicyclo[3,1,1]hept-3-yl, 1,5-Dimethyl-3-aza-bicyclo[3,1,1]hept-3-yl, 7-Aza-bicyclo[2,2,1]hept-7-yl, 2-Aza-bicyclo[3,2,1]oct-2-yl, 3-Aza-bicyclo[3,2,1]oct-3-yl, 8-Aza-bicyclo[3,2,1]oct-8-yl, 3-Aza-bicyclo[3,2,2]non-3-yl oder 3-Aza-bicyclo [4,2,2]dec-3-yl bedeutet und alk für $C_2$-$C_4$-Alkylen der Formel $-CH_2)_n$- steht, wobei n 2, 3 oder 4 bedeutet, und ihrer Salze.

8. Verfahren gemäss Anspruch 1 oder 2 zur Herstellung von 3-(1,5-Dimethyl-3-aza-bicyclo[3,1,1]hept-3-yl)-1-hydroxy-propan-1,1-diphosphonsäure oder eines Salzes davon.

9. Verfahren gemäss Anspruch 1 oder 2 zur Herstellung von 3-(3-Aza-bicyclo[3,2,2]non-3-yl)-1-hydroxy-propan-1,1-diphosphonsäure oder eines Salzes davon.

10. Verfahren gemäss Anspruch 1 oder 2 zur Herstellung von 3-(1,3,3-Trimethyl-6-aza-bicyclo[3,2,1]oct-6-yl)1-hydroxy-propan-1,1-diphosphonsäure oder eines Salzes davon.

11. Verfahren zur Herstellung von pharmazeutischen Präparaten, dadurch gekennzeichnet, dass man eine Verbindung, erhältlich gemäss einem der Ansprüche 1 bis 10, mit üblichen pharmazeutischen Hilfsstoffen vermischt.

Europäisches Patentamt

# EUROPÄISCHER RECHERCHENBERICHT

Nummer der Anmeldung

EP 88 81 0751

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | EP-A-0 170 228 (BOEHRINGER MANNHEIM GmbH)<br>* Ansprüche *<br>--- | 1,10-15 | C 07 F 9/65<br>A 61 K 31/675 |
| P,Y | EP-A-0 272 208 (CIBA-GEIGY AG)<br>* Ansprüche *<br>----- | 1,10-15 | |
| | | | RECHERCHIERTE SACHGEBIETE (Int. Cl.4) |
| | | | C 07 F 9/00 |

Der vorliegende Recherchenbericht wurde für alle Patentansprüche erstellt

| Recherchenort | Abschlußdatum der Recherche | Prüfer |
|---|---|---|
| DEN HAAG | 30-01-1989 | BESLIER L.M. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument